# EUROPEAN PATENT APPLICATION

(11) **EP 4 400 033 A1**
(43) Date of publication of application: **17.07.2024**
(21) Application number: 23151720.2
(22) Date of filing: 16.01.2023
(51) Int. Cl.: A61B 5/00

(54) **MEDICAL IMAGING**

(71) Applicant: Koninklijke Philips N.V., 5656 AG Eindhoven (NL)
(72) Inventor: SENEGAS, Julien Thomas, Eindhoven (NL); FRERKING, Lena Christina, Eindhoven (NL)
(74) Representative: Philips Intellectual Property & Standards

(57) **Abstract**

The invention provides a method for configuring the positioning and/or boundaries of a scan range (volume-to-be-scanned) of a subject based on processing camera data with a prediction model to predict positions of bones structures within the body, and then determining the scan range based on reference to the bone structure positions. The bone structure positions provide spatial reference points or anchor points for defining the scope of the volume-to-be-scanned.

## Description

### FIELD OF THE INVENTION

The present invention relates to a method for configuring a medical imaging scan, in particular to configuring the spatial boundaries of a volume-to-be-scanned.

### BACKGROUND OF THE INVENTION

Proper positioning of the target anatomy of a patient with respect to a medical imaging system (e.g. Computed Tomography, MRI) is of particular importance to ensure optimal image quality for subsequent diagnostic analysis, in order to optimize signal-to-noise ratio (SNR) and, for ionizing modalities, radiation dose distribution.

The positioning includes positioning within a horizontal plane, which typically defines the field-of-view of the scan, and positioning vertically, which ensures proper centering of the target anatomy.

In this context, 'positioning' is used to refer to the positioning of the volume-to-be-scanned by the scanner, i.e. the volumetric scan window, or scan field of view, so as to encompass a particular target anatomy. In practice, this scan window is implemented by physically positioning the patient relative to the scanner, either statically or with a certain motion path, so that the correct volume of the patient is scanned. Since the target anatomy is internal to the subject, its position relative to the coordinate system of the scanner cannot be directly detected. Traditionally, it was necessary for a radiographer to use their experience to make a best estimate as to the placement and boundaries of the scanning window.

Within the current state of the art, the selection of horizontal and vertical positions of the patient can be automated by means of sensor-based approaches where the position of the target anatomy is computed by processing the sensor data, i.e. typically camera images. However, high accuracy models are needed to predict the position of the target anatomies based on the sensor data. This makes the models highly sensitive to any errors in input data or processing. Also, the models currently known in the art are very inflexible with regards to customizing the scan volume positioning. These models provide fixed definitions of scan window start and end points for a given input sensor dataset. However, it would be of value to enable greater customization.

Improvements in this area of the technical field are generally sought. Prediction models for spine, head/neck, and body applications, such as chest, cardiac, abdomen, liver, and pelvis, are of high value since these are important and routine clinical imaging applications.

### SUMMARY OF THE INVENTION

The invention is defined by the claims.

According to examples in accordance with an aspect of the invention, there is provided a method for use in set-up of an imaging scan to be performed using an imaging scanner, the imaging scanner having an examination area for receiving a subject to be scanned;
obtaining spatial sensor data of an outside of a body of the subject received within the examination area;
applying a pre-determined algorithm to the sensor data to determine a position, relative to a co-ordinate system associated with the examination area, of one or more anatomical landmarks of the body of the subject located within the examination area;
applying a prediction procedure, wherein the prediction procedure comprises application of a prediction algorithm adapted to generate an output comprising a prediction of a position within the examination area relative to said co-ordinate system, of a plurality of bone structures of a subject within the examination area, based on an input to the prediction algorithm comprising a detected position within the examination area of the one or more anatomical landmarks;
defining a positioning and/or boundary points of a scanning volume to be scanned relative to said co-ordinate system based on the predicted positions of the bone structures output from the prediction algorithm;
generating an output indicative of the defined positioning and/or boundary points of a scanning volume to be scanned.

The general concept of this invention is to provide an automated method for determining a volume-to-be-scanned (or scan window), but wherein an intermediate stage is introduced in which, from sensor data, positions of bone structures are predicted, and then the start and end points and positioning of the volume-to-be-scanned are determined with reference to the bone structure positions. Bone structure positions provide a very useful set of reference points, internal of the patient, relative to which the volume-to-be-scanned can be determined. Bone structures are useful reference points because the various anatomies which are typically scanned can in most cases be defined in their positioning very accurately relative to bones. The positioning of bony structures of the skeleton relative to organs is very predictable, and this makes skeletal features useful and reliable reference points.

The one or more anatomical landmarks detected using the sensor data are for example landmarks visible of the outside of the body, i.e. landmarks whose locations are observable at a surface of the body, for example joints, or externally visible organs or portions thereof, such as eyes or ears, and so on. Thus, the positions of such anatomical landmarks could be positions defined on the surface of the body, or could be positions within the body estimated based on observations at the surface of the body.

In come embodiments, the plurality of bone structures comprises a plurality of spine vertebrae.

Using the spine as a reference for anatomical positions has the advantage that it provides a natural registration of human anatomies into a relative, size-invariant coordinate system and it nicely correlates with the joint structures such as shoulders and hips that are typically detected on camera images.

For instance, according to one preferred set of embodiments, the defining the positioning and/or boundary points of the scanning volume can comprise: obtaining a first indication of the position and/or boundary points, the first indication defined relative to positions of one or more of the bone structures; and converting the first indication to a second indication of the position and/or boundary points, the second indication defined relative to said co-ordinate system, the converting comprising use of the predicted positions of the bone structures output from the prediction algorithm.

The method may comprise explicitly defining a body co-ordinate system using the detected spine positions as reference points.

The first indication (relative to the bone structures) can be obtained from a user interface. The first indication can additionally or alternatively be obtained from a reference database.

The aforementioned co-ordinate system may be a co-ordinate system of the imaging scanner. This means for example a co-ordinate system relative to which positions operative imaging components of the scanner are defined, and/or relative to which an imaging field of view is defined. It is the co-ordinate system relative to which the scan region acquired by the scanner is defined for example.

In some embodiments, the method comprises determining a position and/or spatial extent of one or more target anatomies within the body, relative to the co-ordinate system, based on the predicted positions of the bone structures.

In some embodiments, the determining the position and/or spatial extent of the one or more target anatomies within the body comprises: obtaining a first indication of the position and/or spatial extent, the first indication defined relative to positions of one or more of the bone structures; and converting the first indication to a second indication of the position and/or spatial extent, the second indication defined relative to said co-ordinate system, the converting comprising use of the predicted positions of the bone structures output from the prediction algorithm. Defining said positioning and/or boundary points of the scan volume may then (optionally) be performed based on the determined position and/or spatial extension of the one or more target anatomies.

For example, it may comprise: obtaining a first indication of the positioning and/or boundary points, the first indication defined relative to positions of the one or more target anatomies; and converting the first indication to a second indication of the positioning and/or boundary points, the second indication defined relative to said co-ordinate system, the converting comprising use of the determined position and/or spatial extent of the one or more target anatomies.

In some embodiments, the method comprises determining a vertical center of the spatial extent of one or more target anatomies within the body, relative to the co-ordinate system, based on the predicted positions of the bone structures, and aligning a vertical center of the scan volume with said vertical center of the target anatomy in the body.

For example, said co-ordinate system may include a vertical dimension, and wherein the said determining the position and/or spatial extent of the one or more target anatomies within the body includes determining a vertical center of a spatial extent of at least one target anatomy within the body, relative to said vertical dimension of the co-ordinate system.

In some embodiments, the positioning and/or boundary points of the scan volume is determined such as to align a vertical center of the scan volume with said vertical center of the at least one target anatomy in the body.

In some embodiments, the imaging scanner is a CT imaging scanner having a rotating gantry which rotates in a plane of rotation. In some embodiments, the imaging scanner is an MRI scanner.

As a general principle, in some embodiments, the imaging scanner has an imaging isocenter defining a central point of the scanning volume to be scanned in at least one dimension, and wherein the scanner implements scanning of a given scanning volume by changing a relative positioning of an operative scanning section of the scanner relative to a subject being scanned, either by moving the scanning section, or by moving a subject support carrying a subject. For example, for a CT scanner, the operative scanning section could be the rotating gantry which carries the x-ray emitter and detector. For an MRI scanner, it could be coil arrangement.

In some embodiments, the method comprises controlling a patient support table of an imaging scanner to adjust a vertical positioning of the patient support table, defined relative to said vertical dimension of the co-ordinate system, such that the vertical center of a target anatomy within the body is aligned with the imaging isocenter of the scanner.

In some embodiments, the method comprises determining a vertical center of the spatial extent of one or more target anatomies within the body, relative to the said co-ordinate system. The vertical dimension of the co-ordinate system corresponds to a vertical direction of the scanner. For a CT scanner, this might be a radial dimension of said plane of rotation of the gantry for example.

With reference to the prediction algorithm, in some embodiments, the prediction algorithm may be a statistical model, such as a regression model.

In some embodiments, the prediction algorithm may be a trained machine learning algorithm. A machine-learning algorithm is any self-training algorithm that processes input data in order to produce or predict output data. Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

In some embodiments, the aforementioned spatial sensor data comprises camera data. This might be video camera data. It could include in some examples 3D camera data. However this is not essential, and other examples might include 2D camera data. Camera data might include infrared camera data or visible-spectrum camera data. An alternative to camera data might include laser sensing spatial sensing data, such as LIDAR data.

In some embodiments, the aforementioned one or more anatomical landmarks include one or more skeletal joints.

In some embodiments, the prediction procedure comprises application of a plurality of prediction algorithms, one for each of the plurality of bone structures whose positions are to be predicted.

In some embodiments, the spatial sensor data is data acquired using a set of one or more sensor elements, e.g. cameras, having pre-defined spatial positions relative to the co-ordinate system referred to earlier. For example, the set of sensor elements may include a spatial array of sensor elements, for example a 2D array, for example arranged in a grid formation.

In some embodiments, the one or more target anatomies include one or more of: a section of the spine, the head or neck, the chest, the heart or a portion thereof, the abdomen, the liver, the pelvis.

Another aspect of the invention is a computer program product comprising code means configured, when run on a processor, to cause the processor to perform a method in accordance with any embodiment or example described in this document, or in accordance with any claim of this application.

The invention can also be embodied in hardware form. An aspect of the invention is a processing unit for use in set-up of an imaging scan to be performed using an imaging scanner, the imaging scanner having an examination area for receiving a subject to be scanned. It is proposed that the processing unit comprise: an input/output; and one or more processors. The one or more processors are adapted/configured/programmed to execute the following steps:
receive at the input/output spatial sensor data of an outside of a body of the subject received within the examination area;
apply a pre-determined algorithm to the sensor data to determine a position, relative to a co-ordinate system associated with the examination area, of one or more anatomical landmarks of the body of the subject located within the examination area;
apply a prediction procedure, wherein the prediction procedure comprises application of a prediction algorithm adapted to generate an output comprising a prediction of a position within the examination area relative to said co-ordinate system, of a plurality of bone structures of a subject within the examination area, based on an input to the prediction algorithm comprising a detected position within the examination area of one or more anatomical landmarks;
define a positioning and/or boundary points of a scanning volume to be scanned relative to said co-ordinate system based on the predicted positions of the bone structures output from the prediction algorithm; and
generate at the input/output an output indicative of the defined positioning and/or boundary points of a scanning volume to be scanned.

As another aspect of the invention, a system can be provided, comprising: a processing unit as set out above, or in accordance with any embodiment in this disclosure or any claim; and an imaging scanner comprising an examination area for receiving a subject.

In some embodiments, the imaging scanner is a computed tomography (CT) scanner or an MRI scanner. In some embodiments, the imaging scanner is an x-ray imaging scanner.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a schematic outline of an example CT scanner known in the art;
Fig. 2 is a block diagram of steps of an example method in accordance with one or more embodiments of the invention;
Fig. 3 is a block diagram of components of an example processing unit and system in accordance with one or more embodiments of the invention;
Fig. 4 schematically illustrates the capturing of sensor data of the outside of the subject and identification of landmark positions;
Fig. 5 illustrates a real-world example of captured sensor data and identified landmark positions;
Fig. 6 illustrates predictions of vertebrae positions based on the identified landmark positions; and
Fig. 7 illustrates accuracy of a regression model used to predict scan ranges by comparing ground truth to model outputs for different predictions.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a method for configuring the positioning and/or boundaries of a scan range or window (volume-to-be-scanned) of a subject based on processing sensor data such as camera data with a prediction model to predict positions of bone structures such as spine vertebrae within the body, and then determining the scan range based on reference to the bone structure positions. The bone structure positions provide spatial reference points or anchor points for defining the scope of the volume-to-be-scanned.

In practice, the particular volume-to-be-scanned is implemented by physically positioning the patient relative to the scanner, either statically or with a certain motion path, so that the correct volume of the patient is scanned. The scanner has a local or native 3D co-ordinate system and the relative position of an operative scanning part/section of the scanner and a patient supporting part of the scanner can be adjusted relative to this 3D co-ordinate system, by moving the scanning part, the patient supporting part, or both. Purely by way of example, in a CT scanner, the scanning part would be the emitter-detector structure which is rotatably supported by a rotating gantry, and this gantry is linearly moveable along an axial direction relative to the patient support table (usually by physically moving the table).

Thus, if the correct scan volume to be scanned can be determined within the 3D coordinate system of the scanner, then the scan volume can be correctly scanned by the standard scan control procedures already encoded in the control software of the scanner which utilizes the native scanner co-ordinate system.

Current approaches for automated positioning of a patient, to achieve a particular scanned region, rely on fixed definitions of target anatomies relative to the 3D co-ordinate system of the scanner, typically based on clinical guidelines. This includes for example the definition of the isocenter of the localizer/survey scan (for MRI), or start and end points of the localizer scan (for CT), based on external landmarks, such as chin, shoulders, hips, or internal landmarks, such as lungs, liver, pelvic bones, etc. Statistical models, e.g. regression, or machine learning (e.g. deep learning) models are then trained to compute the specified positions based on the input sensor data. However, very often clinical institutions have local preferences to define the center or start and end positions of the scans for each anatomy. Technical operators may also need to adapt these preferences for a particular patient if a clinician referral asks for a particular scan. For these reasons, automated positioning using fixed, pre-learned anatomical landmarks applies only to very standard examinations and may not be suitable for all cases. This is particularly true for the case of the spine, for which the start and end points of the corresponding scans can vary frequently depending on the clinical case.

Thus an improved approach is needed. Although reference is made to a localizer scan, embodiments of this invention are applicable to any kind of medical imaging scan.

Embodiments of this invention propose an improved approach in which positions of bone structures such as spine vertebrae, pelvic bones, skull, shoulder bones or any other bone feature or structure are predicted, and these are used as reference points for defining the planned scanning volume. Use of spine vertebrae as the predicted bone structures represents one particularly advantageous example for instance.

Embodiments of this invention are applicable to a wide range of medical imaging modalities, including for example Computed Tomography imaging (CT), Magnetic Resonance Imaging (MRI), X-ray imaging, and PET imaging.

To aid understanding of the descriptions of the inventive concepts which will follow, there will now be briefly described the basic structure of an example CT scanner. However, it is emphasized that the invention is not limited to use with this particular construction of CT scanner, or indeed to CT imaging at all.

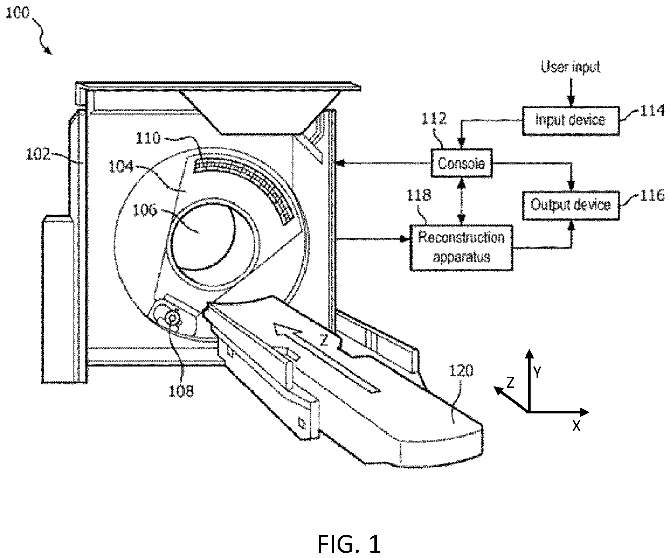

Fig. 1 illustrates an imaging system 100 such as a computed tomography (CT) scanner.

The imaging system 100 includes a generally stationary gantry 102 and a rotating gantry 104. The rotating gantry 104 is rotatably supported by the stationary gantry 102 and rotates around an examination region about a longitudinal or z-axis.

A patient support 120, such as a couch, supports an object or subject such as a human patient in the examination region. The support 120 is configured to move the object or subject for loading, scanning, and/or unloading the object or subject. The patient position can be adjusted in all three dimensions: horizontally (X-Z plane according to the example co-ordinate system shown in Fig. 1), and vertically (Y axis, according to the example co-ordinate system shown in Fig. 1).

A radiation source 108, such as an x-ray tube, is rotatably supported by the rotating gantry 104. The radiation source 108 rotates with the rotating gantry 104 and emits radiation that traverses the examination region 106.

A radiation sensitive detector array 110 subtends an angular arc opposite the radiation source 108 across the examination region 106. The detector array 110 includes one or more rows of detectors that extend along the z-axis direction, detects radiation traversing the examination region 106, and generates projection data indicative thereof.

A general-purpose computing system or computer serves as an operator console 112 and includes an input device(s) 114 such as a mouse, a keyboard, and/or the like and an output device(s) 116 such as a display monitor, a filmer or the like. The console 112 allows an operator to control operation of the system 100.

A reconstruction apparatus 118 processes the projection data and reconstructs volumetric image data. The data can be displayed through one or more display monitors of the output device(s) 116.

The reconstruction apparatus 118 may employ a filtered-backprojection (FBP) reconstruction, a (image domain and/or projection domain) reduced noise reconstruction algorithm (e.g., an iterative reconstruction) and/or other algorithm. It is to be appreciated that the reconstruction apparatus 118 can be implemented through a microprocessor(s), which executes a computer readable instruction(s) encoded or embed on computer readable storage medium such as physical memory and other non-transitory medium. Additionally or alternatively, the microprocessor(s) can execute a computer readable instruction(s) carried by a carrier wave, a signal and other transitory (or non, non-transitory) medium.

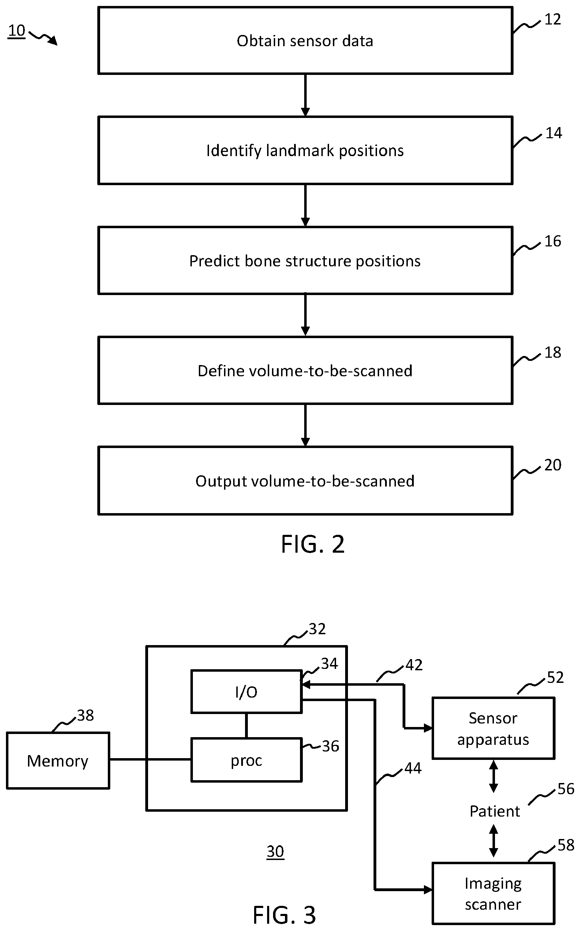

Fig. 2 outlines in block diagram form steps of an example method according to one or more embodiments. The steps will be recited in summary, before being explained further in the form of example embodiments.

The method 10 is for use in set-up of an imaging scan to be performed using an imaging scanner. The imaging scanner has an examination area for receiving a subject to be scanned.

The method comprises obtaining 12 spatial sensor data of an outside of a body of the subject received within the examination area. For example, the spatial sensor data may be camera data, for example image data, for example 3D camera data.

The method further comprises applying a pre-determined algorithm to the sensor data to determine 14 a position, relative to a co-ordinate system associated with the examination area, of one or more anatomical landmarks of the body of the subject located within the examination area.

The co-ordinate system can be a co-ordinate system of the imaging scanner. It may be a co-ordinate system of a reference frame of the imaging scanner for example. It could be a co-ordinate system of a reference frame of the patient support table 120.

The one or more anatomical landmarks can for instance include one or more skeletal joints, such as shoulders or hips.

The method further comprises applying a prediction procedure, wherein the prediction procedure comprises application of at least one prediction algorithm adapted to generate an output comprising a prediction 16 of a position relative to said co-ordinate system, of a plurality of bone structures of a subject within the examination area, based on an input to the prediction algorithm comprising a detected position within the examination area of the one or more anatomical landmarks. Thus the landmark positions are mapped to bone structure positions. The at least one prediction algorithm may for example comprise application of a statistical model such as a regression algorithm, or may comprise a trained machine learning algorithm.

The method further comprises defining 18 a positioning and/or boundary points of a scanning volume to be scanned relative to said co-ordinate system based on the predicted positions of the bone structures output from the prediction algorithm. The scanning volume to be scanned might otherwise be referred to a scan region, a scan window, or a scan field of view.

The method may further comprise generating 20 an output indicative of the defined positioning and/or boundary points of a scanning volume to be scanned.

As noted above, the method can also be embodied in hardware form, for example in the form of a processing unit which is configured to carry out a method in accordance with any example or embodiment described in this document, or in accordance with any claim of this application.

To further aid understanding, Fig. 3 presents a schematic representation of an example processing unit 32 configured to execute a method in accordance with one or more embodiments of the invention.

The processing unit 32 comprises an input/output 34, and one or more processors 36. The processing unit 32 is adapted to receive at the input/output 34 spatial sensor data of an outside of a body of the subject received within the examination area. For example, this may be received from a sensor apparatus 52 communicatively coupled with the input/output. The processing unit 32 is further adapted to apply a pre-determined algorithm to the sensor data to determine a position, relative to a co-ordinate system associated with the examination area, of one or more anatomical landmarks of the body of the subject located within the examination area. The processing unit 32 is further adapted to apply a prediction procedure, wherein the prediction procedure comprises application of a prediction algorithm adapted to generate an output comprising a prediction of a position within the examination area relative to said co-ordinate system, of a plurality of bone structures of a subject within the examination area, based on an input to the prediction algorithm comprising a detected position within the examination area of one or more anatomical landmarks. The processing unit 32 is further adapted to define positioning and/or boundary points of a scanning volume to be scanned relative to said co-ordinate system based on the predicted positions of the bone structures output from the prediction algorithm. The processing unit 32 is further adapted to generate at the input/output 34 an output indicative of the defined positioning and/or boundary points of a scanning volume to be scanned. By way of example, this output may be transferred to an imaging scanning 58 which may be communicatively coupled with the input/output.

The processing arrangement might further comprise a memory 38, which might store computer-executable instructions (computer program code) which, when run by the one or more processors 36, causes the one or more processors to perform the method outlined above.

Also an embodiment of the invention is a system which comprises the processing arrangement 32 in combination with one or both of the sensor apparatus 52 and the imaging scanner 58.

By way of example, the imaging scanner may be a computed tomography scanner or an MRI scanner.

In some embodiments, the plurality of bone structures referred to previously are a plurality of spine vertebrae. This represents a particularly advantageous example because the spine provides a natural registration of human anatomies into a relative, size-invariant coordinate system. The spine may be particularly advantageous when imaging anatomical structures in the upper body.

For imaging anatomical structures in the lower body, or in the extremities of the upper body, or in the head, other bone structures might be useful. For example, the bone structures could include the pelvic bone(s), the skull (or sections thereof), shoulder bones, arm bones, or any other bony structure.

The bone structures could include a combination of spine vertebrae and other bone structures in some examples.

As a general principle, in some embodiments, the imaging scanner may have an imaging isocenter defining a central point of the scanning volume to be scanned in at least one dimension, and wherein the scanner implements scanning of a given scanning volume by changing a relative positioning of an operative scanning section of the scanner relative to a subject being scanned, either by moving the scanning section, or by moving a subject support carrying a subject. For example, for a CT scanner, the operative scanning section could be the rotating gantry which carries the x-ray emitter and detector. For an MRI scanner, it could be coil arrangement.

In some embodiments, the method comprises determining a vertical center of the spatial extent of one or more target anatomies within the body, relative to the co-ordinate system, based on the predicted positions of the bone structures, and aligning a vertical center of the scan volume with said vertical center of the target anatomy in the body.

For example, said co-ordinate system may include a vertical dimension, and wherein the said determining the position and/or spatial extent of the one or more target anatomies within the body includes determining a vertical center of a spatial extent of at least one target anatomy within the body, relative to said vertical dimension of the co-ordinate system.

In some embodiments, the positioning and/or boundary points of the scan volume is determined such as to align a vertical center of the scan volume with said vertical center of the at least one target anatomy in the body.

This might be implemented for example by controlling a patient support table of the imaging scanner to adjust a vertical positioning of the patient support table, defined relative to said vertical dimension of the co-ordinate system, such that the vertical center of a target anatomy within the body is aligned with the imaging isocenter of the scanner.

With regards to the previously mentioned one or more spatial sensor elements, preferably these are implemented with one or more cameras.

In some embodiments, the spatial sensor data is data acquired using a set of one or more sensor elements, e.g. cameras, having pre-defined spatial positions relative to the co-ordinate system referred to earlier.

For example, the set of sensor elements may include a spatial array of sensor elements, for example a 2D array, for example arranged in a grid formation. In some embodiments, the method may include a calibration procedure in which a positioning, and preferably orientation, of each of the set of sensor elements relative to the co-ordinate system is determined. This could be achieved for example by capturing a respective sensor dataset, e.g. an image, using each sensor element, identifying in each dataset a location of a same set of fiducial points within the imaged examination area, the fiducial points each having a known location relative to the co-ordinate system, and using the detected fiducial point locations within each dataset to determine a positioning of each sensing element, e.g. each camera.

One preferred approach to implementing the method outlined in summary above is to define the positioning and/or boundary points of the scanning volume in two stages: first obtaining a first indication of the position and/or boundary points, where the first indication is defined relative to positions of one or more of the bone structures, and second to convert the first indication to a second indication of the position and/or boundary points, the second indication defined relative to the co-ordinate system of the scanner, the converting comprising use of the predicted positions of the bone structures output from the prediction algorithm.

The first indication could be an indication obtained from input by a user received at a user interface.

By way of one example, a user interface may comprise a display unit, and wherein the display unit is controlled to display an image or avatar of a human body, or to display a medical image (2D or 3D, e.g. an MRI or CT image),and wherein the positions of the bones structures are indicated, and wherein the boundaries of the desired scan volumes can be interactively selected by the user relative to the displayed image or avatar. For example, the user controls a pointer device to spatially indicate the position and/or boundaries of the desired scan volume relative to the displayed positions of the bone structures.

The first indication could alternatively be pre-stored in a reference database and simply retrieved, e.g. reflecting a preferred placement of the scanning volume relative to bone structures which is set on a policy level by a medical institution or clinic, or configured in advance by an individual clinician.

Additionally or instead of obtaining an indication of the intended scanning volume relative to the bone structure positions, the method can comprise obtaining a first indication of the position and/or spatial extent of one or more target anatomies, the first indication defined relative to positions of one or more of the bone structures; and converting the first indication to a second indication of the position and/or spatial extent, the second indication defined relative to said co-ordinate system, the converting comprising use of the predicted positions of the bone structures output from the prediction algorithm.

The positioning and/or boundary points of the scan volume can then be determined for example based on the determined position and/or spatial extension of the one or more target anatomies. Again, the first indication can be obtained from a user, or from a datastore.

An example implementation of the method which follows the above approach, and is in accordance with one or more embodiments of the invention, will now be described by way of illustration of the above-summarized concept of the invention. It will be appreciated that not all features of this particular set of embodiments are essential to the inventive concept, and are described to aid understanding and to provide an example to illustrate the inventive concepts.

By way of general introduction, according to this set of embodiments, it is proposed to use a prediction algorithm that predicts the horizontal and vertical positions of at least a subset of the spine vertebrae (e.g. C1 to C6, T1 to T12, L1 to L5, S1 to S5, coccyx) based on externally observable landmarks such as head, shoulders, and hips that are extracted from sensor data. Thus, in this case, the bone structures which have previously been referred to are spine vertebrae. Detection for example of landmarks such as externally observable joints or other anatomical features using camera images has been an active field of research in computer vision. For example, segmentation or classification algorithms are known for performing such tasks, and the skilled person will be aware of means to implement this feature.

From the anatomical landmark positions, one or more prediction algorithms can be applied to predict 3D positions of the vertebrae.

By way of example, suitable prediction algorithms include statistical models such as regression models, or trained machine learning algorithms. A machine-learning algorithm is any self-training algorithm that processes input data in order to produce or predict output data. Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

For example, in the training stage of the regression model or other machine learning model, the ground truth can be provided in the form of a set of 3D images of the whole or part of the spine which are annotated to label the 3D positions of the vertebrae and 3D positions of the externally detectable anatomical landmarks. From this, either one or a plurality of regression models could be trained to predict the position of each of the vertebrae (as output) based on the position of the anatomical landmarks (as input).

The clinical users of the system then have the possibility to define the end and start points and/or the isocenters of the target anatomies and/or of the volumetric scan window (volume to be scanned) based on any one or more vertebrae.

This is useful especially for head / neck, spine, and body applications, such as chest, cardiac, abdomen, liver, and pelvis.

By way of brief summary, according to a particular set of embodiments, the steps of the method may include the following.

Accessing and applying a regression model (or other statistical model) to predict the 3D positions of at least a subset of a subject's vertebrae based on an input indicative of 3D positions of a plurality of externally observable skeletal joints or other landmarks. As already mentioned, the model(s) can be trained based on suitably annotated volumetric MRI or CT image data.

A user interface of the system can be used by a clinical user to pre-define and store in a datastore, or to select in real time start and end points (and/or a center) of scan region to be scanned, or an anatomy to be scanned, potentially on the fly, relative to one or more of the vertebrae, such as T1 to T12 for a thoracic spine scan. It is noted that the scan region to be scanned may optionally be a scan region of a localizer or survey scan, based on which a final selection of the scan region is determined. The scan region in any embodiment may be a 2D or 3D scan region.

In operation, during a set-up of the patient examination, sensor data, preferably camera images, of the patient positioned for the examination are acquired. An algorithm for the detection of the landmarks, such as one or more skeletal joints or other anatomical features from the sensor data is applied to obtain the coordinates of the landmarks relative to a co-ordinate system. The aforementioned vertebra regression model is applied to predict patient-specific vertebra positions. The clinical operator selects the start and end points (and/or the center) of the scan region (e.g. localizer scan region) based on the desired vertebrae.

In the case of a spine scan, the optimal vertical center of the selected spine segment is computed based on the vertebra regression model for example.

This vertebrae-based approach gives the clinical operator full freedom to define the proper position based on clear anatomical reference points (the vertebrae), as opposed to standard approaches in which only a pre-defined set of positions can be selected from the system.

Using the spine as reference for anatomical positions has the advantage that it provides a natural registration of human anatomies into a relative, size-invariant coordinate system and it correlates well with the joint structures such as shoulders and hips that are typically detected on camera images.

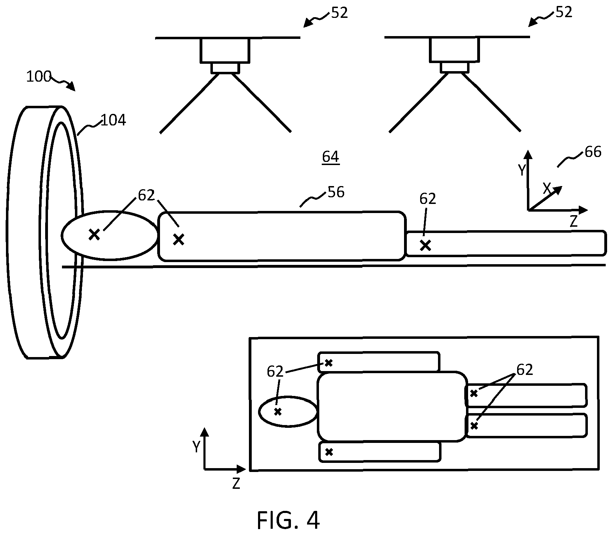

In accordance with any embodiment of this invention, a part of the method is the obtaining of sensor data of the subject, the identification of anatomical landmarks, and the prediction of vertebrae positions. These steps of the method will now be discussed in further detail with reference to Figs. 4-6. The features to be described can be applied to any embodiment of this invention.

Fig. 4 schematically illustrates the step of acquiring the spatial sensor data using a sensor apparatus 52. Fig. 4 (left) shows an elevation view. Fig. 4 (bottom) shows a plan view. The sensor apparatus in the illustrated example comprises a plurality of cameras, e.g. 3D cameras. Just a single camera could instead be used. Use of cameras is not essential. Other examples may include laser-based detection, non-camera optical detection, use of electromagnetic sensing and so on.

In some embodiments, the spatial sensor data comprises camera data, for example 3D camera data. 3D camera image data refers depth images. These can be acquired with either a depth camera / 3D sensor, e.g. a time-of-flight camera, or a pair stereo sensors. With this 3D data, the outer contour of the body can be represented. By taking into account also data of an empty table, the thickness of a specified region can be calculated, which can for example be used for finding a vertical center.

Fig. 4 shows a patient 56 positioned in an examination area 64 of an imaging apparatus 100. For illustration, this is shown as a CT scanner, and the rotatable gantry 104 is indicated. Other modalities can be used instead.

From the sensor data, the locations within a co-ordinate system 66 of the examination area 64 of a plurality of anatomical landmarks 62 are determined. The landmarks may be a plurality of joints or other visible anatomical structures, such as shoulders, head, and hips, for example. This is shown schematically in the plan view of Fig. 4 (bottom). In this example, head, left and right shoulders, as well as left and right hips have been detected.

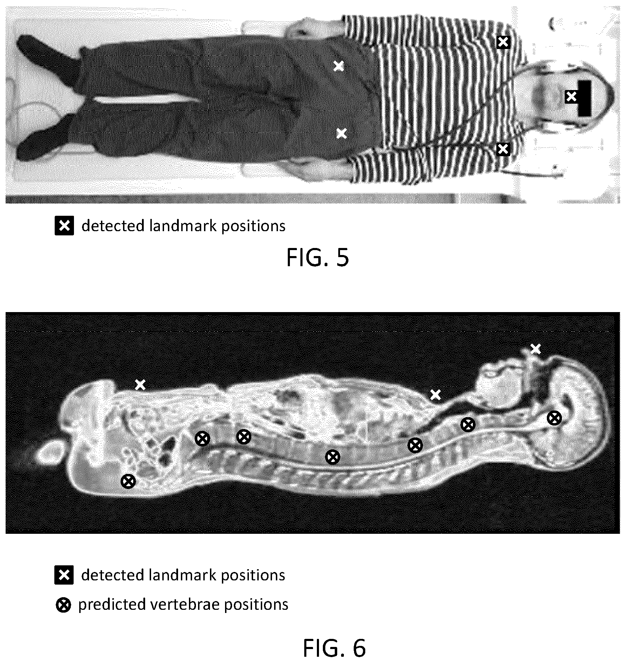

Fig. 5 shows a real-world example of spatial sensor data in the form of an image of a subject, with the locations of the detected landmarks indicated.

By applying an algorithm, such as a regression model, the locations of at least a subset of the subject's vertebrae can be detected.

Fig. 6 schematically illustrates predicted positions of vertebrae of the spine of a subject based on already detected positions of anatomical landmarks. The positions are shown projected on an MRI dataset for the patient.

A part of the method is the prediction of vertebrae positions using an algorithm such as a regression model or machine learning model.

As noted above, the model can be trained based on multiple annotated datasets comprising sensor data of the same format and modality as the sensor apparatus 52 which will be used in operation, the sensor data being annotated with both the anatomical landmark positions and the vertebrae positions. From this, the model can be trained to predict the vertebra positions based on the landmark positions.

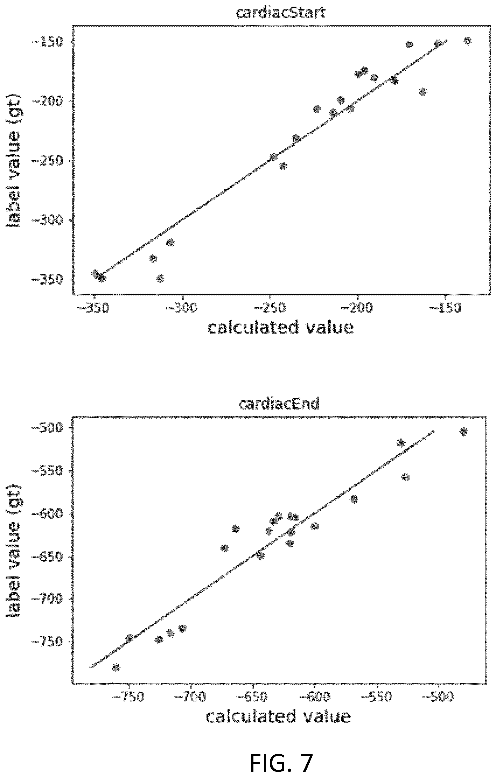

By way of illustration, Fig. 7 shows the accuracy of a pair of example regression models that were trained in this way for predicting vertebra locations C6 (Fig. 7, top) and L3 (Fig. 7, bottom). These also correspond to the start and end points respectively (along the z-axis using the co-ordinate system already introduced above) of an ideal cardiac scanning region, for imaging the heart. Each graph shows the correlation between the output of the model and the ground truth (gt). It can be seen that the correlation is very strong, showing good prediction results.

By training regression models for each single vertebra for instance, every clinician is able to select the size of a scan region individually, e.g. based on the guidelines of a clinic. These selected areas can then be used to first calculate the vertical center within this region and, based on that, for example perform a surview scan for the target anatomy.

In some embodiments, a further regression / machine learning model can be applied which is adapted to predict boundaries and/or a positioning of a scan volume to be scanned, for capturing a specific anatomy of interest, based either on predicted positions of the vertebrae (already predicted using a first one or more prediction models), or based directly on the obtained spatial sensor data. For the latter case, such a model could be trained based on training data comprising a plurality of spatial sensor datasets being annotated with the landmark positions and being annotated with the ideal boundaries and/or central positioning of a volume to be scanned for capturing a particular anatomy.

It is noted although preferred embodiments use a regression model to predict the vertebrae positions, other algorithm types can instead be used, such as other statistical models, or a machine learning or artificial intelligence algorithm trained in a similar way as already described above for the regression model.

This invention can be applied for a wide range of different image scanning technologies, including, by way of non-limiting example, MRI, CT, or DXR (Digital X-ray radiogrammetry).

It is further noted that although the embodiment discussed above refers to predicting positions of a plurality of vertebrae, the sample principles can be applied equally to predict the positions of other bony structures such as the pelvic bones, the skull or sections thereof, the shoulder bones and so on. The acquired sensor data (e.g. camera data) is able to detect visibly observable anatomical landmarks, but not internal structures. Thus the general concept is one of using the sensor-detected landmarks to predict locations of internal bone structures, which can then be used effectively as a set of anchoring points relative to which a scan region or scanner positioning can be defined, for instance by predicting locations of an internal anatomical feature relative to the bone structures.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing arrangement can be implemented. The processing unit includes a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method for use in set-up of an imaging scan to be performed using an imaging scanner, the imaging scanner having an examination area for receiving a subject to be scanned;
obtaining spatial sensor data of an outside of a body of the subject received within the examination area;
applying a pre-determined algorithm to the sensor data to determine a position, relative to a co-ordinate system associated with the examination area, of one or more anatomical landmarks of the body of the subject located within the examination area;
applying a prediction procedure, wherein the prediction procedure comprises application of a prediction algorithm adapted to generate an output comprising a prediction of a position within the examination area relative to said co-ordinate system, of a plurality of bone structures of a subject within the examination area, based on an input to the prediction algorithm comprising a detected position within the examination area of the one or more anatomical landmarks;
defining a positioning and/or boundary points of a scanning volume to be scanned relative to said co-ordinate system based on the predicted positions of the bone structures output from the prediction algorithm; and
generating an output indicative of the defined positioning and/or boundary points of a scanning volume to be scanned.

2. The method of claim 1, wherein the plurality of bones structures comprises a plurality of spine vertebrae.

3. The method of claim 1 or 2, wherein the defining the positioning and/or boundary points of the scanning volume comprises:
obtaining a first indication of the position and/or boundary points, the first indication defined relative to positions of one or more of the bone structures;
converting the first indication to a second indication of the position and/or boundary points, the second indication defined relative to said co-ordinate system, the converting comprising use of the predicted positions of the bone structures output from the prediction algorithm.

4. The method of claim 3, wherein
the first indication is obtained from a user interface; or
the first indication is obtained from a reference database.

5. The method of any of claims 1-4, further comprising determining a position and/or spatial extent of one or more target anatomies within the body, relative to the co-ordinate system, based on the predicted positions of the bone structures.

6. The method of claim 5, wherein determining the position and/or spatial extent of the one or more target anatomies within the body comprises:
obtaining a first indication of the position and/or spatial extent of the one or more target anatomies, the first indication defined relative to positions of one or more of the bone structures; and
converting the first indication to a second indication of the position and/or spatial extent of the one or more target anatomies, the second indication defined relative to said co-ordinate system, the converting comprising use of the predicted positions of the bone structures output from the prediction algorithm.

7. The method of claim 5 or 6, wherein defining said positioning and/or boundary points of the scan volume is performed based on the determined position and/or spatial extension of the one or more target anatomies.

8. The method of any of claims 5-7, wherein said co-ordinate system includes a vertical dimension, and wherein the said determining the position and/or spatial extent of the one or more target anatomies within the body includes determining a vertical center of a spatial extent of at least one target anatomy within the body, relative to said vertical dimension of the co-ordinate system, and wherein the positioning and/or boundary points of the scan volume is determined such as to align a vertical center of the scan volume with said vertical center of the at least one target anatomy in the body.

9. The method of any of claims 1-8, wherein the one or more anatomical landmarks include one or more joints.

10. The method of any of claims 1-9, wherein the prediction procedure comprises application of a plurality of prediction algorithms, one for each of the plurality of bone structures whose positions are to be predicted.

11. The method of any of claim 1-10, wherein the spatial sensor data is data acquired using a set of one or more sensor elements, e.g. cameras, having pre-defined spatial positions relative to said coordinate system.

12. The method of any of claims 1-11, wherein the one or more target anatomies include one or more of: a section of the spine, the head or neck, the chest, the heart or a portion thereof, the abdomen, the liver, the kidneys, the stomach, the prostate, the bladder, the pelvis.

13. A computer program product comprising code means configured, when run on a processor, to cause the processor to perform a method in accordance with any of claims 1-12.

14. A processing unit for use in set-up of an imaging scan to be performed using an imaging scanner, the imaging scanner having an examination area for receiving a subject to be scanned, the processing unit comprising:
an input/output; and
one or more processors adapted to:
receive at the input/output spatial sensor data of an outside of a body of the subject received within the examination area;
apply a pre-determined algorithm to the sensor data to determine a position, relative to a co-ordinate system associated with the examination area, of one or more anatomical landmarks of the body of the subject located within the examination area;
apply a prediction procedure, wherein the prediction procedure comprises application of a prediction algorithm adapted to generate an output comprising a prediction of a position within the examination area relative to said co-ordinate system, of a plurality of bone structures of a subject within the examination area, based on an input to the prediction algorithm comprising a detected position within the examination area of one or more anatomical landmarks;
define a positioning and/or boundary points of a scanning volume to be scanned relative to said co-ordinate system based on the predicted positions of the bone structures output from the prediction algorithm;
generate at the input/output an output indicative of the defined positioning and/or boundary points of a scanning volume to be scanned.

15. A system comprising:
a processing unit as claimed in claim 14; and
an imaging scanner comprising an examination area for receiving a subject.
